# FASCICULE DE BREVET EUROPEEN

(11) **EP 0 882 788 B1**
(45) Date de publication et mention de la délivrance du brevet: **01.03.2006**
(21) Numéro de dépôt: 98401361.5
(22) Date de dépôt: 08.06.1998
(51) Int. Cl.: C12N 5/06, C12N 5/08

(54) **Procédé de séparation de plaquettes ou de microparticules d'origine plaquettaire à partir d'un échantillon les contenant**
Verfahren zur Trennung von Blutbestandteilen oder aus Blutplätchen Mikropartikel aus einem diese enthaltenten Muster
Process for the separation of platelets or microparticles from microplatelets from a sample containing them

(30) Priorité: 06.06.1997 FR 9707032
(43) Date de publication de la demande: 09.12.1998
(73) Titulaire: Biocytex, 13010 Marseille (FR)
(72) Inventeur: Poncelet, Philippe, 13010 Marseille (FR); Besson-Faure, Isabelle, 13001 Marseille (FR)
(74) Mandataire: Warcoin, Jacques

(56) Documents cités:
- EP-A- 0 176 080
- EP-A- 0 350 851
- US-A- 3 997 442
- GADOL N ET AL: "A new method for separating mononuclear cells from whole blood." DIAGNOSTIC IMMUNOLOGY, (1985) 3 (3) 145-54, XP002057791
- PONCELET ET AL JOURNAL OF IMMUNOLOGICAL METHODS vol. 85, no. 1, 1985, pages 65 - 74

## Description

La présente invention concerne la séparation de plaquettes ou de microparticules d'origine plaquettaire à partir d'un prélèvement biologique les contenant.

L'invention s'applique aussi bien aux plaquettes qu'aux micro-particules d'origine plaquettaire. Par micro-particule d'origine plaquettaire, on entend une sous unité, ou vésiculation de la cellule obtenue par tout traitement, mécanique ou autre ou encore résultant d'un état d'activation cellulaire ou d'un état physiopathologique particulier, tel que l'apoptose ou la lyse cellulaire ou plaquettaire par exemple.

Dans la suite de la description, le terme « cellules » sera utilisé pour désigner indifféremment, sauf indication contraire, les différents produits que l'on souhaite séparer, notamment les plaquettes et les micro-particules d'origine plaquettaire.

La présente invention a comme objectif spécifique de séparer d'un échantillon les molécules liées à des cellules, des mêmes molécules non liées présentes dans la phase liquide, notamment sous forme soluble. Ces molécules peuvent être par exemple des marqueurs cellulaires ou des éléments liés en surface des cellules, tels que des anticorps, ou des ligands. Dans certains cas, il peut être intéressant de doser à la fois l'élément lié à des cellules et l'élément non lié présent dans la phase liquide, notamment sous forme soluble, ou encore le rapport des deux.

Lorsque l'on effectue les dosages de marqueurs cellulaires dans les échantillons sanguins, il est nécessaire de séparer les cellules du milieu qui portent ces marqueurs des mêmes marqueurs présents à l'état soluble et donc susceptibles de fausser les dosages ultérieurs.

La méthode la plus communément utilisée pour séparer les cellules consiste à centrifuger l'échantillon après un éventuel traitement préalable.

On obtient ainsi un culot contenant les cellules et sur lequel sont fixées les molécules liées, les molécules non liées restant dans un surnageant (qui peut être éliminé par toute méthode, notamment par aspiration/décantation).

Toutefois, lors de la centrifugation, le culot cellulaire a tendance à emprisonner des molécules non liées, il est donc nécessaire de remettre le culot en suspension à plusieurs reprises afin d'effectuer des lavages pour réduire significativement la concentration résiduelle des molécules non liées. Ainsi, et de façon caractéristique, il est nécessaire d'effectuer au moins trois lavages avec une dilution au 1/10 à chaque étape (100 µl résiduel dilués à 1ml) pour réduire de 3 log (1000 fois) la concentration résiduelle de molécules non liées.

Ces centrifugations répétées ont bien entendu de nombreux inconvénients parmi lesquels :
- une perte de cellules ;
- une fragilisation des cellules ;
- une formation d'agrégats entre les cellules qui sont parfois difficilement réversibles, il s'agit en particulier des cellules qui ont tendance notamment à adhérer entre elles et en particulier les plaquettes ;
- la longueur de la procédure et la lourdeur de la manipulation. En effet, trois lavages même avec une centrifiguation courte (5 mn) nécessitent en tout près de 30 mn de traitement ;
- les liaisons sont instables ; les liaisons de type ligand/récepteur, anticorps/antigène, enzyme/substrat sont réversibles et la dissociation des molécules liées est d'autant plus marquée que le temps de lavage est prolongé. Les temps de lavage longs représentent donc un défaut du procédé d'autant plus marqué que l'affinité de la paire de liaison est faible (vitesse de dissociation rapide). Dans ce cas il devient indispensable de :
   - raccourcir le temps de lavage ;
   - réduire le volume de phase liquide résiduelle en contact avec les molécules liées aux cellules, mais ce qui réduit d'autant l'efficacité du lavage.

Enfin on note dans le cas de manipulations de matériels infectieux, la formation d'aérosols qu'il est bien entendu souhaitable d'éviter si cela est possible.

Dans le cas particulier des plaquettes, les centrifugations répétées induisent en plus des problèmes cités ci-dessus, une agrégation et une activation qui sont toujours très préjudiciables aux dosages ultérieurs et qui, en tout état de cause modifient les propriétés biologiques des plaquettes après les différentes étapes de centrifugation.

C'est pourquoi, depuis longtemps on a tenté d'apporter des modifications au procédé connu afin de remédier à certains des inconvénients cités précédemment.

Il a en particulier été proposé de faire des centrifugations sur coussin de sérum, qui sont moins traumatisantes pour les cellules mais qui en fait sont évidemment beaucoup plus chères et surtout plus délicates à mettre en oeuvre, ce qui fait que ce type de procédé n'a été que peu utilisé.

On a également envisagé les centrifugations au travers d'une phase huileuse hydrophobe (voir notamment P. Poncelet and P. Carayon, J. Immunol. Methods (1985), 85, 65-74 et Titus JA et al., Proc. Natl. Acad. Sci. USA, 78, 519). Cette stratégie, qui est surtout utilisée pour les mesures de radio-immunologie, présente un certain nombre d'avantages. Elle est extrêmement efficace pour séparer les molécules libres qui restent dans la phase aqueuse et les molécules liées qui sont entraînées dans la phase hydrophobe avec les cellules ; la séparation est rapide, de l'ordre de 2 mn et la concentration résiduelle de molécules non liées, moins de 1%. Il y a en outre très peu de dissociation de molécules liées dans le temps après séparation.

Il faut toutefois noter que lorsque l'on souhaite soumettre les cellules ainsi séparées à une cytométrie de flux, à une microscopie ou à des tests d'activité biologique ou encore à des remises en culture, cette technique évidemment défaut que les cellules, une fois passées dans l'huile, ne sont plus utilisables. De plus, dans le cas des plaquettes, le contact avec une huile minérale génère une activation/agrégation qui bien entendu les rend totalement impropres à tout traitement postérieur.

Il a également été envisagé des lavages sur supports filtrants, par exemple par dépression. L'avantage est que dans ce cas on a moins de manipulation. On peut traiter les grandes séries et ceci est facilement adaptable aux « screening », les lavages sont efficaces et rapides, ce qui est avantageux, le principal défaut est la non disponibilité des cellules (récupération exclue) pour des analyses telles que pré-citées.

Des procédés assez généraux, qui concernent, le lavage de cellules par centrifugation, ont été décrits dans la technique antérieure. Par exemple, EP 0 176 080 et Gadol N. et al (Diagnostic Immunology, 1985 3 (3) 145-154) portent sur des méthodes de lavage de populations cellulaires mononuclées grâce à un gel qui apparaît peu traumatisant pour les cellules. D'autres techniques à base de gel hydrophobe sont décrites dans EP 176 080, Poncelet et al, Journal of Immunological Methods, 1985, Vol. 85, No 1 pages 65-74 at Titus et al, Proc. Natl. Acad. USA, 1981, Vol. 78, No 1, pages 519-523. US 3 997 442 porte sur une méthode pour séparer deux phase de densités différentes à partir d'un fluide à partir d'un matériau thixotropique déposé au fond du tube de centrifugation.

Enfin, la séquence centrifugation sur coussin ou gradient d'albumine + chromatographie sur gel de tamisage moléculaire a déjà été proposée pour éliminer les molécules plasmatiques non liées aux plaquettes. Toutefois cette technique de laboratoire qui est utilisée pour éliminer dans certains cas les protéines plasmatiques non liées aux plaquettes et susceptibles d'interagir avec des tests fonctionnels, reste très longue et complexe à mettre en oeuvre.

C'est pourquoi la présente invention propose un procédé de séparation des plaquettes ou des micro-particules d'origine plaquettaire à partir d'un échantillon les contenant caractérisé en ce que :
a) on centrifuge ledit échantillon dans un récipient contenant à son extrêmité distale un gel compact, ledit gel étant sensiblement imperméable à la phase liquide de l'échantillon mais pouvant être pénétré dans les conditions de centrifugation par les plaquettes ou micro-particules d'origine plaquettaires à séparer ;
b) on sépare éventuellement le surnageant de centrifugation
c) et on récupère ensuite les plaquettes ou micro-particules d'origine plaquettaires incorporées dans le gel ; ledit gel étant constitué de particules hydrophile qui à l'état gonflé ont un diamètre compris entre 30 et 500 µm et de préférence, compris entre 90 et 350 µm.

Les plaquettes ou micro-particules d'origine plaquettaire incorporées dans le gel sont ensuite récupérées par mise en suspension du gel dans un milieu approprié tel que par exemple un milieu tampon et/ou à force ionique particulière. Puis on sépare les plaquettes ou micro-particules d'origine plaquettaire du gel par exemple par filtration. Ceci permet d'obtenir une suspension plaquettaire qui peut ainsi être concentrée par rapport à l'échantillon de départ ; le procédé de la présente invention peut donc être également un procédé de concentration.

Ce procédé repose sur le fait que lors d'une centrifugation, la capacité de pénétration des éléments solides contenus dans une solution est directement proportionnelle à leur masse, ainsi, les particules les plus lourdes vont être capables de pénétrer dans le gel ou même éventuellement de le traverser, alors que les molécules solubles de même que le milieu liquide, ne seront dans la plupart des cas, pas capables de pénétrer le gel correspondant.

Bien entendu, le procédé selon la présente invention devra pour chaque séparation, être adapté à la nature des produits, en particulier aux plaquettes, que l'on souhaite séparer en agissant sur la nature et les propriétés physiques du gel ainsi que sur les vitesses de centrifugation afin d'assurer une séparation des phases telle que souhaitée.

Ce procédé est particulièrement adapté à la séparation, à partir d'un échantillon les contenant, des molécules liées à des cellules, des mêmes molécules non liées, présentes dans la phase liquide, notamment sous forme soluble.

Le gel mis en oeuvre dans le procédé selon la présente invention est de préférence un gel hydrophile constitué de particules qui à l'état gonflé, ont un diamètre compris entre 30 et 500 µm, et de préférence compris entre 90 et 350 µm,

De même, lorsqu'il est en place, le gel gonflé a des caractéristiques telles que la concentration en poids sec des particules est comprise entre 0,04 et 0,2 g/ml.

Bien entendu, le gel en question peut être prêt à l'emploi, c'est-à-dire déjà placé au fond du récipient de centrifugation ou bien être mis en place de façon extemporanée, c'est-à-dire à partir d'un gel dilué qui sera sédimenté par centrifugation.

Ce gel sera, de façon préférentielle, choisi parmi la gamme Sephadex®, notamment les gels commercialisés sous la désignation G25, G100, G200 ou équivalent, couramment utilisés pour la chromatographie de tamisage moléculaire.

La vitesse de centrifugation sera de préférence telle que la force centrifuge soit comprise entre 200 g et 5000 g.

Ainsi, de façon schématique, le procédé comprend ;
1) l'introduction d'un échantillon liquide qui contient notamment les plaquettes ou les micro-particules d'origine plaquettaire dans un tube de centrifugation au dessus d'un système gélifié ;
2) une centrifugation à une vitesse appropriée ;
3) le lavage par élimination, notamment aspiration ou décantation de la phase liquide ;
4) la récupération des plaquettes ou des micro-particules d'origine plaquettaire dans le gel ou comme on le verra ci-après éventuellement sous le gel.

Le procédé selon la présente invention permet donc d'obtenir deux phases distinctes :
- un surnageant au dessus du gel qui contient l'ensemble des molécules restées en solution, dans certains cas l'excès de réactifs, mais dans d'autres cas les protéines solubles cellulaires qui ne sont pas restées fixées sur les cellules, et ;
- un gel qui contient notamment les plaquettes ainsi que les molécules qui sont fixées auxdites plaquettes.

De façon préférentielle, on préfèrera utiliser à titre de récipient de centrifugation un tube dont l'extrêmité distale présente un diamètre plus faible, le gel étant situé dans cette partie distale de diamètre plus faible. Par « partie distale » ou « extrémité distale » on entend bien entendu désigner la partie la plus éloignée de l'ouverture du tube. Le tube de centrifugation comportant ainsi un rétreint dans sa partie inférieure, permet de concentrer les cellules d'une part et d'autre part lorsque le gel est préparé un certain temps avant l'utilisation, il est assuré d'une meilleure conservation. C'est pourquoi en général, le diamètre de la zone de gel, c'est-à-dire le diamètre de la partie inférieure du tube sera de préférence compris entre 2 et 7 mm, et de préférence inférieur à 5 mm. De façon générale, le rapport des volumes de la chambre supérieure, c'est-à-dire l'échantillon ou le surnageant et la chambre de collecte, est préférentiellement supérieur à 10, permettant dans ces conditions une meilleure concentration.

Il est également possible de prévoir dans un mode de réalisation préféré, que la partie distale du tube dans lequel est placé le gel soit ouverte à son extrêmité, cette ouverture étant obturée par un filtre susceptible de retenir le gel. Dans ce cas ladite extrémité ouverte débouche dans un récipient qui doit être accouplé de façon amovible au tube de façon à recevoir les éléments figurés de l'échantillon ayant traversé le gel.

Il convient de rappeler que les cellules migrent en fonction de leur masse et qu'en principe elles restent emprisonnés dans le gel. Dans ce cas, on peut récupérer les cellules contenues dans le gel en remettant le gel en suspension, puis on filtre la suspension avec un filtre qui retient les particules de gel et autorise le passage des éléments figurés. Cependant, dans le cas où l'on augmente la force de centrifugation ou bien lorsque l'on augmente la durée de la centrifugation, ou par utilisation d'un gel approprié, il est possible de faire migrer ces cellules au travers du gel. Dans ce cas, elles sont susceptibles de passer directement dans le récipient inférieur. Bien entendu dans le cas où on utilise un tel dispositif, il convient de remplir le récipient inférieur avec un liquide avant la centrifugation.

Le récipient de la partie inférieure peut être notamment un tube vissé sur la partie inférieure du tube de centrifugation proprement dit ou bien accouplé par tous moyens appropriés. Dans ce mode de mise en oeuvre, il est donc possible de prévoir dans le récipient inférieur, l'établissement d'un gradient correspondant à deux ou plus de densités, ce qui permettra en même temps lors de la centrifugation de séparer les différentes espèces cellulaires en fonction de leur densité. La méthode permettant de réaliser les gradients de densité est connue et ne sera pas redécrite en détails ici. La plupart du temps, on pourra se contenter d'un gradient à deux densités qui permettra par exemple de séparer les plaquettes des autres éléments figurés du sang.

Parmi les échantillons qui peuvent être traités selon le procédé selon la présente invention, il faut citer plus particulièrement :
- un plasma riche en plaquettes ;
- le sang total, de préférence préalablement traité par une solution de type lyse des erythrocytes ;
- une suspension de cellules dans une solution équivalente au tampon de récupération ou différente, comme par exemple :
   - une solution de fixation (paraformaldéhyde, formol, glutaraldéhyde, éthanol) ;
   - une solution de lyse différentielle (lyse des erythrocytes) ;
   - une solution de traitement enzymatique (phospholipase, trypsine, protéinase...) chimique (acide formique, citrique, acétique...), solutions préférées si les cellules doivent impérativement être séparées de la solution après arrêt/neutralisation par dilution ;
   - une solution contenant un réactif, immunologique (anticorps, antigène) ou non (ligand), substrat d'enzyme, élément d'une paire de fixation (tel que biotine, X-biotine, avidine...)

Comme cela a été mentionné précédemment, il est particulièrement intéressant d'utiliser des suspensions dans lesquelles les cellules ont été traitées par un réactif. En effet, dans ce cas, le réactif en excès pourra être séparé par le procédé selon la présente invention et il sera possible d'envisager la révélation du réactif ou même d'ailleurs du marqueur des cellules lorsque cela est possible par introduction d'un élément révélateur dans le gel.

Ainsi il est possible d'envisager l'incorporation dans le gel d'un anticorps anti-IgG de souris marqué, par exemple fluorescent, à l'aide de produits tels que le DDAF (fragment F(ab')₂ d'anticorps anti-IgG de souris conjugué au FITC, commercialisé par la société Eurobio), puis d'introduire à la partie supérieure du tube un milieu contenant des cellules ainsi qu'un anticorps monoclonal de souris réactif avec lesdites cellules. Afin que la réaction soit complète, l'anticorps monoclonal sera bien entendu en excès et dans ces conditions, une partie des anticorps sera fixée sur les cellules, les autres resteront dans la solution. Après traitement à l'aide du procédé selon la présente invention, les cellules marquées par les anticorps monoclonaux vont pénétrer dans le gel, et au niveau de ce gel elles vont être reconnues par les anticorps anti-IgG de souris fluorescents et donc amener une révélation.

L'un des avantages de ce procédé, est qu'il est possible de prévoir des tubes prétraités contenant le gel et l'anticorps anti-IgG de souris fluorescent, le manipulateur n'ayant plus alors qu'à réaliser l'étape de fixation des anticorps monoclonaux puis la centrifugation, la révélation apparaissant indirectement au niveau du gel.

En outre, la séparation des molécules non liées permet d'utiliser un réactif de seconde couche en quantité très faible, ce qui évidemment élimine du bruit de fond au niveau de l'immuno-marquage et améliore donc la sensibilité.

On peut également dans la même approche, prévoir la mise en évidence, par exemple d'un marqueur de surface cellulaire notamment lorsqu'il s'agit d'enzymes. Cette enzyme doit se présenter aussi bien à l'état lié qu'à l'état non lié. Si le gel contient le substrat de ladite enzyme avec un chromogène ou un fluorogène après la centrifugation, on pourra immédiatement visualiser dans le gel la présence de cellules porteuses du marqueur enzymatique.

Parmi les enzymes utilisables, il faut citer notamment les ecto-enzymes cellulaires et solubles à la fois comme les enzymes de la chaîne de coagulation portées par des leucocytes, des plaquettes ou des micro-particules issues de plaquettes, (par exemple les facteurs Xa, XIa, IXa et la protéine C). Il peut également s'agir d'enzymes purement cellulaires mais dont un inhibiteur ou un substrat gênant le test est présent sous forme soluble dans le plasma.

Enfin, il peut s'agir d'enzymes couplées à un réactif, par exemple anticorps, ligand, avidine conjugué à péroxydase, β-galactosidase, acetylcholine estérase.

Dans toutes ces opérations, la centrifugation permet d'éliminer les espèces solubles non liées aux cellules.

De nombreuses caractéristiques et avantages de la présente invention apparaîtront à la lecture des exemples ci-après.

### EXEMPLE 1 : Mesure des Ig associées aux plaquettes :

Les immunoglobulines (Ig) sont normalement présentes dans le plasma à 10-20 mg/ml. La présence de cette quantité très élevée rend obligatoire, avant immuno-marquage par un anticorps anti-Ig, le lavage extensif des plaquettes présentes dans le plasma riche en plaquettes (PRP), i.e. 2 à 4 lavages, par centrifugation.

Ces centrifugations répétées entraînent :
- Des pertes importantes qui nécessitent des échantillons de départ de grande taille (jusqu'à 5-10 ml de sang), incompatibles avec des tests en pédiatrie néo-natale ;
- Les plaquettes ne peuvent pas être lavées par centrifugation en présence des globules rouges et globules blancs, ce qui oblige à travailler sur un PRP (une centrifugation initiale supplémentaire) ;
- Des risques importants de décrochage et perte de tout ou partie (donc biais de sélection) des Ig anticorps anti-plaquettes, réputées être de faible affinité (auto-anticorps) ;
- Une charge de travail et un temps d'occupation des centrifugeuses (chaque lavage réclame une centrifugeuse rapide pendant 15 mn) non négligeables.

Dans la technique tube-gel, selon la présente invention, l'échantillon peut être indifféremment du sang total ou du PRP.

Le surnageant est décanté par simple retournement (opération répétée 3 fois = 3 lavages). Le gel, contenant les cellules (plaquettes +/- globules rouges et globules blancs) est remis en suspension dans un faible volume de tampon d'immuno-marquage.

Le gel est retenu sur les mailles d'un filtre (grille nylon ouverture 35 µm) tel que, par exemple le « Cell-Strainer » de Falcon/Becton-Dickinson et les cellules récupérées après centrifugation courte (3 min, 800 rpm) en vue de l'immuno-marquage.

On dispose alors d'une suspension de plaquettes (+/globules rouges et globules blancs), dépourvue de plasma et en particulier des Ig plasmatiques, qui ont conservé leurs caractéristiques morphologiques permettant l'analyse par cytométrie en flux, leur phénotype immunologique initial, qui n'ont pas perdu d'anticorps associés au cours d'opérations de lavage, et qui n'ont pas été activées par le procédé de séparation.

L'efficacité du « lavage » réalisé par le transfert dans le gel a été testée en utilisant une Ig traceur fluorescente, qui est dosée dans l'échantillon de départ et dans la suspension finale. On a mesuré un facteur de dilution >> à 1000 entre concentration initiale et finale du traceur. Ceci correspond, pour un plasma contenant 10 mg/ml d'Ig à une concentration résiduelle ≤ 10 µg/ml d'Ig dans la suspension de cellules. Une telle concentration résiduelle réclamerait au minimum 2 à 3 lavages des plaquettes dans les conditions citées ci-dessus.

### EXEMPLE 2 : Fixation de plaquettes pour immuno-marquage différé

Pour la mesure des Ig associées aux plaquettes comme pour d'autres antigènes de surface plaquettaires, un protocole de stabilisation des cellules permettant de maintenir à son niveau initial l'expression des antigènes d'intérêt est un problème majeur et délicat.

La résolution de ce problème sur les plaquettes permettrait de différer l'analyse en cytométrie de flux (opération demandant une technique particulière) du traitement de l'échantillon (opération à réaliser sur site dans un délai court).

Une fixation, à la fois douce mais efficace et standardisée, réclame un traitement de l'échantillon cellulaire avec le fixateur soluble d'une durée limitée, avec remplacement du fixateur par un milieu de conservation dépourvu de fixateur.

Le système tube-gel offre cette possibilité.

L'échantillon (FRP, 500 µl) est ainsi dilué dans le fixateur (PFA 0,2 %, 500 µl) et l'incubation poursuivie 10 min.

L'analyse quantitative du phénotype immunologique des plaquettes ainsi fixées et immédiatement débarassées du fixateur après 10 min a démontré la fiabilité de ce protocole pour une conservation d'une semaine des principaux antigènes plaquettaires visés dans des protocoles d'études pharmacologiques multi-centriques/multi-nationaux.

Le procédé selon la présente invention peut être également utilisé à plus grande échelle afin d'effectuer une préparation rapide de plaquettes lavées, ce qui constitue une alternative à la méthode de Mustard (Methods in Enzymology, 1989, Vol. 169, 3-21). D'autre part, certains traitements chimiques ont été décrits sur les plaquettes et les leucocytes (par exemple un traitement à l'acide citrique pH 4) pour décaper les antigènes HLA et détecter spécifiquement les anticorps dirigés contre les antigènes autres que HLA. De tels traitements réclament évidemment une neutralisation avec élimination de certaines molécules chimiques en contact avec les cellules et il est certain que le procédé selon la présente invention qui est particulièrement rapide et simple devrait permettre une mise en oeuvre facilitée de ce type de processus.

De même les traitements enzymatiques peuvent réclamer une séparation rapide des éléments cellulaires (par exemple, trypsine, PIPLC ou Protéinase K). Une centrifugation standard laisse les cellules en contact pendant plus de 5 min avec le surnageant contenant l'enzyme active, alors que grâce au procédé selon la présente invention, on peut envisager de limiter la durée du temps de contact.

Il est également possible de prévoir l'utilisation du procédé selon la présente invention dans la méthode QIFI de quantification de molécules cellulaires par cytométrie qui est basée sur des marquages indirects (en deux couches anticorps monoclonaux 1 + anti Ig fluorescent). Cette méthode est décrite dans Poncelet et al., J. of Immunol. Methods, 85 (1985), 65-74, dont le contenu est ici incorporé pour référence. C'est un principe difficile à appliquer avec un contre marquage (anticorps monoclonal 2 porteur d'un autre fluochrome. La méthode de séparation en éliminant l'excès de réactif anticorps monoclonal 1 et anti Ig permet d'envisager un contre marquage de façon simple à mettre en oeuvre.

De même il est possible d'écarter rapidement certains réactifs non souhaités, comme par exemple des activateurs, des cytokines, des messagers, des produits toxiques à court terme(eau distillée pour la lyse des érythrocites ou l'acide formique), des agents complexants tels que l'EDTA ou le Citrate ou des ions déclenchant des réactions tels que le calcium ou le magnésiuin.

### Exemple 3 : Protocole de séparation

- Préparation d'un plasma riche en plaquettes.
   Le sang total est centrifugé 15 min. à 170 g. La phase supérieure correspondant au PRP (plasma riche en plaquettes) est prélevée.
- Séparation plaquettaire
   - 200 µl de PRP sont ajoutés à 2 ml de tampon approprié au sein du tube gel,
   - centrifugation 5 min. - 1 200 g,
   - élimination de la phase liquide supérieure par retournement,
   - 2 lavages en tampon approprié du gel contenant les plaquettes (ajout de 2 ml de tampon dans le tube, au-dessus du gel et élimination par retournement),
   - mise en suspension du gel et des plaquettes au moyen d'une pipette,
   - transfert de la suspension sur un filtre dans un bouchon de tube,
   - centrifugation du tube bouché, les particules du gel sont retenues par le filtre tandis que les plaquettes "tombent" au fond du tube,
   - élimination du bouchon filtre et recueil du filtrat.

### Exemple 4 : Caractéristiques du gel

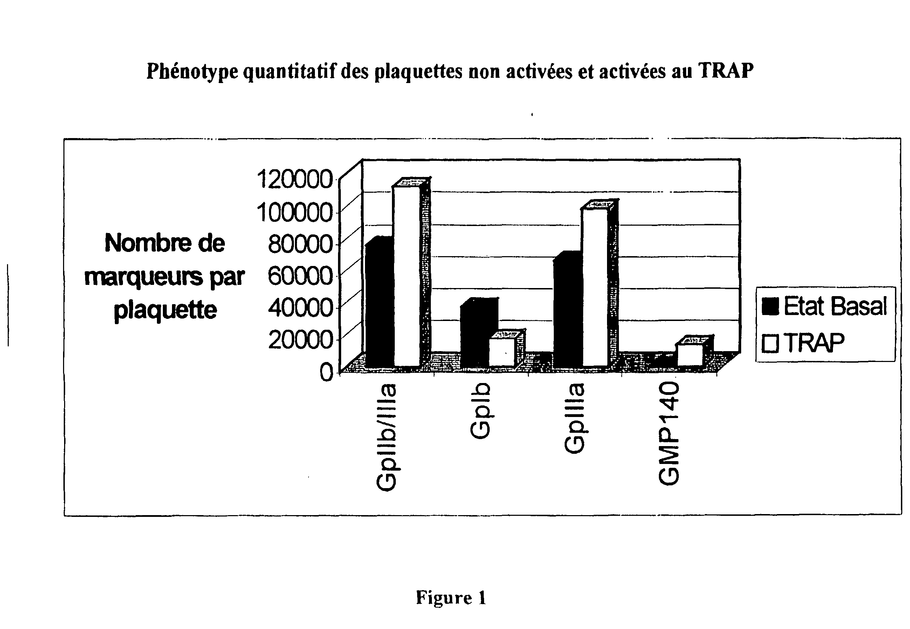

- Rendement : 51% (voir tableau 1 et figure 1, infra)
   - numération des plaquettes dans le filtrat, numération à rapporter au microlitre,
   - comparaison de cette numération avec celle du PRP de départ,
   - établissement d'un rapport numération filtrat/numération PRP exprimé en pourcentage.
- Respect de l'intégrité plaquettaire
   Absence d'activation des plaquettes au cours du processus de séparation (voir tableau 1). Le niveau d'activation plaquettaire est objectivé par la mesure de marqueurs de surface spécifiques.
   Les plaquettes sont considérées non activées lorsque la GMP140 est inférieure à 500 copies par plaquette.
   Respect de la réactivité plaquettaire (tableau 1). Le maintien de la réactivité plaquettaire est défini par les modifications quantitatives de certains marqueurs de surface suite à une activation in vitro. On a choisi :
   - la GMP140 qui ne s'exprime que sur les plaquettes activées,
   - la GpIIb/IIIa dont la valeur augmente lors de l'activation,
   - la GpIb dont la valeur chute lors de l'activation par internalisation.

   La réactivité plaquettaire est conservée pour l'activateur TRAP.

**TABLEAU 1 : CARACTERISTIQUES DES PLAQUETTES SEPAREES EN TUBE GEL**

| | **Etat basal** | **TRAP** |
|---|---|---|
| GpIIbIIIa | 76 355 | 112 206 |
| GpIb | 37 438 | 17 452 |
| GpIIIa | 66 796 | 98 871 |
| GMP140 | 370 | 13 525 |

### Exemple 5 : Utilisation du tube gel dans le cadre des thrombopénies

Le rendement est reproductible quelle que soit la numération plaquettaire. Ainsi, la méthode de séparation est validée pour une numération plaquettaire normale (150 à 400 x 10⁹ plaquettes/litre). Pour récupérer un nombre de plaquettes plus important une extension de volume de la prise d'essai jusqu'au 500 µl est réalisable pour les échantillons thrombopéniques (10 x 10⁹ à 150 x 10⁹ plaquettes par litre de sang), voir tableau 2 ci-dessous.

**TABLEAU 2**

| **Echantillon** | **Prise d'essai de PRP (µl)** | **Quantité de Plaquettes initiale** | **Quantité de plaquettes séparées/lavées** | **Rendement (%)** |
|---|---|---|---|---|
| Normal (200x10⁹ pIt/l | 200 | 10⁸ | 4.10⁷ | 40 |
| Thrombopénie à 10x10⁹ pIt/l | 200 | 4.10⁶ | 1,6.10⁶ | 42 |
| Thrombopénie à 10x10⁹ pIt/l | 500 | 10' | 4,2.10⁶ | 42 |

### Exemple 6 : Application spécifique

Mesure des immunoglobulines associées aux plaquettes (PAIg). Comparaison des techniques de lavage/séparation en tube gel et par centrifugations multiples (voir tableau 3 ci-après). Le procédé de séparation/lavage des plaquettes par centrifugation est susceptible d'activer ces cellules conduisant à un relarguage des Ig internes responsables de résultats par excès. La mesure des PAIg a été effectuée sur un échantillon normal dont les plaquettes ont été lavées au moyen du tube gel ou au moyen de 3 centrifugations. Il existe en effet un nombre de PAIg mesuré supérieur pour le processus de séparation/lavage par centrifugation (tableau 3).

**TABLEAU 3 : COMPARAISON DES TECHNIQUES DE LAVAGE**

| | **LAVAGES GEL Séparation** | **LAVAGES CENTRIFUGATION** |
|---|---|---|
| Quantification PAIg | 1387 PAIg par plaquette | 2407 PAIg par plaquette |

## Revendications

1. Procédé de séparation de plaquettes ou de micro-particules d'origine plaquettaire, à partir d'un échantillon les contenant choisi parmi un plasma riche en plaquettes et un échantillon contenant des molécules liées à des plaquettes ou des microparticules d'origine plaquettaire et des même molécules non liées présentes dans la phase liquide, notamment sous forme soluble, comprenant les étapes suivantes :
a) on centrifuge ledit échantillon dans un récipient contenant à son extrêmité distale un gel compact, ledit gel étant sensiblement imperméable à la phase liquide de l'échantillon mais pouvant être pénétré dans les conditions de centrifugation par les plaquettes ou les micro-particules d'origine plaquettaire ;
b) on sépare éventuellement le surnageant de centrifugation, et ;
c) on récupère ensuite les plaquettes ou les micro-particules d'origine plaquettaire incorporées dans le gel ;
**caractérisé en ce que** le gel est constitué de particules hydrophiles qui à l'état gonflé ont un diamètre compris entre 30 et 500 µm et de préférence, compris entre 90 et 350 µm.

2. Procédé selon la revendication 1, **caractérisé en ce que** les molécules sont des immunoglobulines.

3. Procédé selon l'une des revendications 1 à 2, **caractérisé en ce que** le gel est présent à une concentration en poids sec de particules de 0,04 g/ml à 0,2 g/ml par rapport au gel.

4. Procédé selon l'une des revendications 1 à 3, **caractérisé en ce que** la vitesse de centrifugation est comprise entre 200 et 5000 g.

5. Procédé selon l'une des revendications 1 à 4, **caractérisé en ce que** le récipient de centrifugation est un tube dont la partie distale présente un diamètre plus faible, le gel étant situé dans cette partie distale de diamètre plus faible.

6. Procédé selon la revendication 5 **caractérisé en ce que** le diamètre plus faible est de l'ordre de 2 à 7 mm.

7. Procédé selon l'une des revendications 5 ou 6, **caractérisé en ce que** la partie distale du tube dans lequel est placé le gel est ouverte à son extrémité inférieure, cette ouverture étant obturée par un filtre susceptible de retenir le gel, ladite extrémité ouverte débouchant dans un récipient accouplée de façon amovible au tube de façon à recevoir les cellules de l'échantillon ayant traversé le gel.

8. Procédé selon la revendication 7, **caractérisé en ce que** le récipient contient un gradient de densité.

9. Procédé selon l'une des revendications 1 à 8, **caractérisé en ce que** dans l'étape c) on récupère le gel en le remettant en suspension et on filtre ladite suspension avec un filtre qui retient les particules de gel et autorise le passage des plaquettes ou des micro-particules d'origine plaquettaire.

10. Procédé selon la revendication 1, **caractérisé en ce que** l'échantillon est constitué par une suspension dans laquelle les plaquettes ou les micro-particules d'origine plaquettaire ont été traitées par un réactif.

11. Procédé selon la revendication 10, **caractérisé en ce que** les plaquettes ou les micro-particules d'origine plaquettaire ont été traitées par un anticorps en excès reconnaissant un antigène plaquettaire à titre de réactif.

12. Procédé selon l'une des revendications 9 à 11, **caractérisé en ce que** le gel contient un composant assurant la révélation du réactif ou du marqueur des plaquettes.

## Claims

1. Process for the separation of platelets or platelet-derived microparticles, from a sample containing them, chosen from a platelet-rich plasma and a sample containing molecules bound to platelets or platelet-derived microparticles and the same unbound molecules present in the liquid phase, in particular in soluble form, comprising the following steps:
a) said sample is centrifuged in a container containing a compact gel at its distal end, said gel being substantially impermeable to the liquid phase of the sample but being able to be penetrated under the centrifugation conditions by the platelets or the platelet-derived microparticles;
b) the centrifugation supernatant is optionally separated; and
c) the platelets or the platelet-derived microparticles incorporated into the gel are subsequently recovered,
**characterized in that** the gel consists of hydrophilic particles which, in the swollen state, have a diameter of between 30 and 500 µm, and preferably of between 90 and 350 µm.

2. Process according to Claim 1, **characterized in that** the molecules are immunoglobulins.

3. Process according to either of Claims 1 and 2, **characterized in that** the gel is present at a concentration by dry weight of particles of 0.04 g/ml to 0.2 g/ml relative to the gel.

4. Process according to one of Claims 1 to 3, **characterized in that** the centrifugation speed is between 200 and 5000 g.

5. Process according to one of Claims 1 to 4, **characterized in that** the centrifugation container is a tube, the distal part of which has a smaller diameter, the gel being located in this distal part that has a smaller diameter.

6. Process according to Claim 5, **characterized in that** the smaller diameter is of the order of 2 to 7 mm.

7. Process according to either of Claims 5 and 6, **characterized in that** the distal part of the tube in which the gel is placed is open at its bottom end, this opening being sealed with a filter capable of retaining the gel, said open end running into a container connected in a detachable manner to the tube so as to receive the cells of the sample that have passed through the gel.

8. Process according to Claim 7, **characterized in that** the container contains a density gradient.

9. Process according to one of Claims 1 to 8, **characterized in that**, in step c), the gel is recovered by resuspending the gel and said suspension is filtered with a filter that retains the gel particles and allows the platelets or the platelet-derived microparticles to pass through.

10. Process according to Claim 1, **characterized in that** the sample consists of a suspension in which the platelets or the platelet-derived microparticles have been treated with a reagent.

11. Process according to Claim 10, **characterized in that** the platelets or the platelet-derived microparticles have been treated with an excess of an antibody that recognizes a platelet antigen, as reagent.

12. Process according to one of Claims 9 to 11, **characterized in that** the gel contains a component for revealing the reagent or the marker for the platelets.

## Patentansprüche

1. Verfahren zur Abtrennung von Blutplättchen oder Mikropartikeln Blutplättchen-Ursprungs aus einer sie enthaltenden Probe, die aus einem an Blutplättchen reichen Plasma und einer Probe ausgewählt ist, welche Moleküle, die an Blutplättchen oder Mikropartikel Blutplättchen-Ursprungs gebunden sind, und gleiche, nicht-gebundene Moleküle enthält, die in der flüssigen Phase vorliegen, insbesondere in löslicher Form, umfassend die folgenden Schritte:
a) man zentrifugiert die Probe in einem Behälter, der an seinem distalen Ende ein kompaktes Gel enthält, wobei das Gel für die flüssige Phase der Probe deutlich undurchlässig ist, aber unter den Zentrifugationsbedingungen von den Blutplättchen oder den Mikropartikeln Blutplättchen-Ursprungs penetriert werden kann;
b) man trennt unter Umständen den Zentrifugationsüberstand ab und
c) man gewinnt anschließend die Blutplättchen oder die Mikropartikel Blutplättchen-Ursprungs, die in dem Gel enthalten sind;
**dadurch gekennzeichnet, dass** das Gel aus hydrophilen Teilchen besteht, die im gequollenen Zustand einen Durchmesser zwischen 30 und 500 µm einschließlich und vorzugsweise zwischen 90 und 350 µm einschließlich aufweisen.

2. Verfahren nach Anspruch 1, **dadurch gekennzeichnet, dass** die Moleküle Immunglobuline sind.

3. Verfahren nach einem der Ansprüche 1 oder 2, **dadurch gekennzeichnet, dass** das Gel in einer Konzentration als Teilchen-Trockengewicht von 0,04 g/ml bis 0,2 g/ml vorliegt, bezogen auf das Gel.

4. Verfahren nach einem der Ansprüche 1 bis 3, **dadurch gekennzeichnet, dass** die Zentrifugationsgeschwindigkeit zwischen 200 und 5000 g einschließlich liegt.

5. Verfahren nach einem Ansprüche 1 bis 4, **dadurch gekennzeichnet, dass** der Zentrifugationsbehälter ein Rohr ist, dessen distaler Teil einen geringeren Durchmesser aufweist, wobei das Gel in diesem distalen Teil mit geringerem Durchmesser angeordnet ist.

6. Verfahren nach Anspruch 5, **dadurch gekennzeichnet, dass** der geringere Durchmesser in der Größenordnung von 2 bis 7 mm liegt.

7. Verfahren nach einem der Ansprüche 5 oder 6, **dadurch gekennzeichnet, dass** der distale Teil des Rohrs, in dem das Gel angeordnet ist, an seinem unteren Ende offen ist, wobei diese Öffnung durch einen Filter verschlossen ist, der das Gel zurückhalten kann, wobei das offene Ende in einen Behälter einmündet, der auf lösbare Weise mit dem Rohr verbunden ist, um die Zellen der Probe, die das Gel durchquert haben, aufzunehmen.

8. Verfahren nach Anspruch 7, **dadurch gekennzeichnet, dass** der Behälter einen Dichtegradienten enthält.

9. Verfahren nach einem der Ansprüche 1 bis 8, **dadurch gekennzeichnet, dass** man im Schritt c) das Gel gewinnt, indem man es wieder in Suspension gibt und die Suspension mit einem Filter filtriert, der die Gelteilchen zurückhält und den Durchtritt von Blutplättchen oder Mikropartikeln Blutplättchen-Ursprungs gestattet.

10. Verfahren nach Anspruch 1, **dadurch gekennzeichnet, dass** die Probe aus einer Suspension besteht, in der die Blutplättchen oder die Mikropartikel Blutplättchen-Ursprungs mit einem Reagens behandelt worden sind.

11. Verfahren nach Anspruch 10, **dadurch gekennzeichnet, dass** die Blutplättchen oder die Mikropartikel Blutplättchen-Ursprungs im Überschuss mit einem Antikörper, der ein Blutplättchen-Antigen erkennt, als Reagens behandelt worden sind.

12. Verfahren nach einem Ansprüche 9 bis 11, **dadurch gekennzeichnet, dass** das Gel eine Verbindung enthält, welche den Nachweis des Reagens oder des Markers der Blutplättchen sicherstellt.
